(19) Europäisches Patentamt

European Patent Office

Office européen des brevets

(11) **EP 1 661 933 A1**

(12) **EUROPÄISCHE PATENTANMELDUNG**

| | |
|---|---|
| (43) Veröffentlichungstag:<br>**31.05.2006 Patentblatt 2006/22** | (51) Int Cl.:<br>***C08G 63/672*** *(2006.01)* |

(21) Anmeldenummer: **05025645.2**

(22) Anmeldetag: **24.11.2005**

| | |
|---|---|
| (84) Benannte Vertragsstaaten:<br>**AT BE BG CH CY CZ DE DK EE ES FI FR GB GR HU IE IS IT LI LT LU LV MC NL PL PT RO SE SI SK TR**<br>Benannte Erstreckungsstaaten:<br>**AL BA HR MK YU** | (72) Erfinder: **Koch, Herbert, Dr.**<br>**46348 Raesfeld (DE)** |
| (30) Priorität: **24.11.2004 DE 102004056785** | (74) Vertreter: **Schupfner, Georg**<br>**Müller Schupfner**<br>**Patentanwälte**<br>**Parkstrasse 1**<br>**21244 Buchholz (DE)** |
| (71) Anmelder: **SASOL Germany GmbH**<br>**20537 Hamburg (DE)** | |

(54) **Fließfähige, amphiphile und nichtionische Oligoester**

(57)    Die Erfindung betrifft bei Raumtemperatur fließfähige, amphiphile, nichtionische Oligoester hergestellt durch Umsetzung von Dicarbonsäure-Verbindungen, Polyol-Verbindungen und wasserlöslichen Alkylenoxid-Anlagerungsprodukten, darüber hinaus Wasch- und Reinigungsmittelformulierungen und Konzentrate enthaltend oben bezeichnete Oligoester und die Verwendung der Oligoester als Additiv in Wasch- und Reinigungsmitteln.

**EP 1 661 933 A1**

**Beschreibung**

Gegenstand der Erfindung sind bei Raumtemperatur fließfähige, amphiphile, nichtionische Oligoester hergestellt durch Umsetzung von Dicarbonsäure-Verbindungen, Polyol-Verbindungen und wasserlöslichen Alkylenoxid-Anlagerungsprodukten, darüber hinaus Wasch- und Reinigungsmittelformulierungen und Konzentrate enthaltend oben bezeichnete Oligoester und die Verwendung der Oligoester als Additiv in Wasch- und Reinigungsmitteln.

Moderne Wasch- und Reinigungsformulierungen unterliegen einem ständigen Prozess der Leistungsoptimierung. Dabei gilt grundsätzlich das Prinzip: "Höhere Performance bei gleicher oder niedrigerer Einsatzkonzentration". Hieraus ergibt sich der Wunsch nach leistungssteigernden Additiven, die ihre Wirkung schon bei geringen Einsatzkonzentrationen entfalten. Dabei besteht insbesondere Bedarf an Additiven, die sich durch Multifunktionalität auszeichnen und eine Darreichungsform aufweisen, die eine leichte Verarbeitung in den modernen Wasch- und Reinigungsmitteln gewährleistet.

Es sind eine Vielzahl unterschiedlicher Poly- und Oligoesterverbindungen als sogenannte "Soil-Release-/Soil-Repellent-Polymere" (Schmutzlöse-bzw. Schmutzabweise Polymere) bekannt.

DE-A-1 469 403 beschreibt ein Verfahren zur oberflächenverändemden Behandlung von aus Polyestern abgeleiteten Artikeln. Dabei sind die hergestellten Polyester aus Ethylenterephthalat- (ET) und Polyoxyethylenterephthalat-Einheiten (POET) aufgebaut, wobei die Polyethylenglykole Molgewichte von 1000 bis 4000 aufweisen und ET : POET im Verhältnis 2 bis 6 : 1 eingesetzt werden. Die Beschichtung der Faser erfolgt durch Wärmebehandlung mit dem Polyester bei Temperaturen von ca. 90°C, wodurch das Gewebe eine dauerhafte Oberflächenbehandlung erfährt, die neben einer Wirkung als Schutzschicht auch eine statische Aufladung des Gewebes verhindert.

Die US 3 959 230 offenbaren ET/POET-Polyester mit einem ET zu POET - Verhältnis von 25 : 75 bis 35 : 65, wobei niedermolekulare Polyethylenglykole mit Molgewichten von 300 bis 700 eingesetzt werden und die gewonnenen Polyester Molgewichte von 25000 bis 55000 aufweisen.

Als eine Form der Konfektionierung von Schmutzlösepolymeren beschreibt die DE-A-33 24 258 das Lösen bzw. Dispergieren eines PET/POET-Polyesters mit einem PET : POET Verhältnis von 2 bis 6 zu 1 in einem flüssigen, nichtionischen Tensid und Versprühen dieser Mischung auf einen Builder (FET-Polyethylenterephthalat).

DE-A-40 0,1 415 beansprucht die Darstellung und Verwendung eines Polyesters als vergrauungsinhibierender und schmutzablösender Zusatz zu pulverförmigen und flüssigen Waschmitteln. Die Polyester werden durch Kondensation von mindestens 2 carboxylgruppenenthaltenden Carbonsäuren mit mehrwertigen Alkoholen erhalten. Zusätzlich werden noch alkoxylierte mehrwertige Alkohole eingesetzt, die durch Anlagerung von 5 bis 80 mol C2-/C3- Alkylenoxiden gewonnen werden. Die Produkte zeichnen sich durch eine verbesserte Wirksamkeit und eine bessere Verträglichkeit mit flüssigen und pulverförmigen Waschmittelformulierungen aus.

Aus der EP 0 752 468-A2 sind wasserlösliche Copolymere mit Soil-Release-Eigenschaften bekannt, die als Monomereinheiten Polyethylenglykole und/oder verschlossene Polyethylenglykole, eine oder mehrere aromatische, ggf. sulfonierte Dicarbonsäuren und mindestens 30 mol % eines Polyols mit mindestens 3 Hydroxylgruppen enthalten. Die in der EP 0 752 468-A2 beschriebenen Produkte sind wachs- oder harzartige Feststoffe.

Die EP 0 442 101 B1 offenbart Polyester, die erhältlich sind durch Kondensation von

a.) Carbonsäuren mit mindestens 2 Carboxylgruppen, deren Estern, Anhydriden oder Mischungen

b.) Glycerin, Pentaerythrit, Oligoglycerin und/oder Additionsprodukten von 1 bis 5 mol mindestens eines Alkylenoxids mit 2 bis 3 C-Atomen an 1 Mol der genannten Alkohole, sowie

c.) wasserlöslichen Anlagerungsprodukten von 5 bis 80 mol mindestens eines Alkylenoxids an 1 mol C8- bis C24-Alkohol, C6- bis C18- Alkylphenol oder C8- bis C24- Alkylamin.

Die beschriebenen Polyester sind wachsartig fest und werden als vergrauungsinhibierende und schmutzablösungsfördexnde Zusätze zu pulverförmigen und flüssigen Waschmittelformulierungen eingesetzt.

WO 99/09125-A1 beschreibt amphiphile Polymere auf Basis von Polyestern mit einkondensierten acetalischen Gruppen, die bei Raumtemperatur flüssig sind, sowie ihren Einsatz in Wasch- und Reinigungsmitteln.

Aus DE 198 26 356-A1 sind Oligoester als schmutzablösende Wäschereinigungsmittel bekannt, die durch Polykondensation von

(a) 40 bis 52 mol% einer oder mehrerer Dicarbonsäuren oder deren Ester
(b) 10 bis 40 mol% Ethylenglykol und/oder Propylenglykol
(c) 3 bis 20 mol% Polyethylenglykol
(d) 0,5 bis 10 mol% eines wasserlöslichen Anlagerungsproduktes von 5 bis 80 mol eines Alkylenoxids an 1 Mol C1- bis C24- Alkohol, C6- bis C18- Alkylphenol oder C8- bis C24- Alkylamin sowie
(e) 0,4 bis 10 mol% eines oder mehrerer Polyole mit 3 bis 6 Hydroxylgruppen erhalten werden. Gemäß Beispiel 1 der DE 198 26 356-A1 sind die erhältlichen Oligoester Feststoffe.

Merkmal der bekannten Soil-Release- / Soil-Repellent-Polymere sind ihre Wirksamkeit bei der Faszteinigung, insbesondere bei der Reinigung von Polyester-/Polyamid-Geweben bzw. deren Mischungen mit Baumwolle. Die Wirkungsweise beruht im Wesentlichen auf einer Modifizierung der hydrophoben Faseroberfläche mit Hilfe des hydrophilierenden Polymers.

Der Feuchtigkeitstransport (Wasseradsorption und Saugfähigkeit) wird bei den mit dem Soil-Release-Polymer behandelten hydrophoben Geweben, wie Polyester-, Polyamidgewebe oder deren Mischungen mit Baumwolle, erheblich verbessert. Außerdem verleihen die Polymere den Stoffen antistatische und Gleiteigenschaften, wodurch die Handhabung dieser Fasern in der Textilverarbeitung erleichtert wird. Die Behandlung des Gewebes mit dem Soil-Release-Polymer ist als eine Art Imprägnierung zu verstehen, d.h. das Schmutzlösepolymer verbleibt für mehrere Wascheyclen auf der Faser.

Viele der bekannten Poly-/Oligoester haben den Nachteil, dass sie als Feststoffe vorliegen, deren Einarbeitung in flüssige bzw. gelartige Formulierungen erschwert wird.

Ein weiterer Nachteil der bekannten amphiphilen Oligoester ist ihre geringe physikalische Stabilität in flüssigen oder gelartigen Formulierungen. Dies führt im Wesentlichen zum Absetzen von Feststoffpartikeln in der fertigen Wasch- bzw. Reinigungsmittelformulierung. Zum Teil werden die amphiphilen oligoester als wässrige Dispersionen angeboten mit den Nachteilen eines geringen Aktivgehaltes von kleiner 25%, sowie der Separation von Feststoffpartikeln während der Lagerung der wässrigen Dispersionen. Weitere Nachteile sind die mangelnde chemische Stabilität in flüssigen Formulierungen, bedingt durch alkalische Esterhydrolyse.

Darüber hinaus zeichnen sich die meisten der im Stand der Technik bekannten Polymere dadurch aus, dass sie zwar eine gute Soil-Release- / Soil-Repellent Performance aufweisen, die eigentliche Primärwaschleistung der Waschmittelformulierung jedoch nur mäßig unterstützen.

Aufgabe der vorliegenden Erfindung war die Entwicklung und Bereitstellung von amphiphilen Oligoestern, die insbesondere die Primärwaschleistung der Waschmittelformulierung signifikant erhöhen und sich darüber hinaus durch eine gute physikalische und chemische/hydrolytische Verträglichkeit in den handelsüblichen Flüssigwaschmittelformulierungen auszeichnen.

Diese Aufgabe wird erfindungsgemäß gelöst durch bei Raumtemperatur fließfähige, amphiphile und nichtionische Oligoester, wobei die Oligoester durch Umsetzung, vorzugsweise Polykondensation, herstellbar sind aus:

(A) 15 bis 50 mol%, vorzugsweise 25 bis 40 mol%, einer oder mehrerer Dicarbonsäure-Verbindungen,

(B) 4 bis 70 mol% einer oder mehrerer Diolverbindungen und

(C) 5 bis 60 mol%, vorzugsweise 15 bis 40 mol%, eines oder mehrerer wasserlöslicher Alkylenoxid- Anlagerungsprodukte, herstellbar aus zwei unterschiedlichen Alkylenoxid-Einheiten durch Anlagerung an einem C1- bis C6-Alkohol (oder an einem ein entsprechendes Vorprodukt) im Molverhältnis 4 bis 100 Mol, vorzugsweise 4 bis 50 Mol Alkylenoxid zu 1 Mol Alkohol, wobei das Alkylenoxid-Anlagerungsprodukt eine 2-Blockstruktur aufweist aus einem C2- Alkylenoxid und einem höheren Alkylenoxid ausgewählt aus der Gruppe umfassend C3- Alkylenoxide und C4-Alkylenoxide, insbesondere mit 2 bis 10 oder sogar nur 2 bis 4 eingebauten Propylenoxid- oder. Butylenoxid - Molekülen.

Bevorzugte Ausrührungsfbnnen sind Gegenstand der Unteransprüche oder nachfolgend geschildert.

Die erfindungsgemäßen Oligoester sind bevorzugt so hergestellt bzw. herstellbar, dass das Alkylenoxid-Anlagerungsprodukt durch Anlagerung

• von Propylenoxid und nachfolgend Ethylenoxid, oder
• insbesondere von Ethylenoxid und nachfolgend Propylenoxid, oder
• von Butylenoxid und nachfolgend Ethylenoxid oder
• insbesondere von Ethylenoxid und nachfolgend Butylenoxid

an einen C1- bis C6- Mono-Alkohol, vorzugsweise Methanol, herstellbar ist, vorzugsweise von 2 bis 10 mol, insbesondere 2 bis 4 mol, Propylenoxid bzw. Butylenoxid und 10 bis 20 mol Ethylenoxid.

Selbstverständlich kann der C1- bis C6- Mono-Alkohol als Anlagerungsmolekül jeweils auch durch ein entsprechendes Vorprodukt ersetzt sein, etwa Diethylenglykolmonomethylether, wenn die Alkoxylierung zunächst mit Ethylenoxid erfolgt oder beispielsweise Dipropylenglykolmonomethylether, wenn die Alkoxylierung zunächst mit Propylenoxid erfolgt.

Nach einer bevorzugten Ausführungsform der Erfindung sind die Carbonsäuregruppen der Oligoester mit zumindest einer, vorzugsweise beidseitig mit Propylenoxid-(Block)Einheiten oder der Butylenoxid-(Block)Einheiten verknüpft, was den Verbindungen überraschend eine hohe chemische und insbesondere hydrolytische Stabilität auch in basischer Umgebung und trotzdem eine ausreichende Kompatibilität mit den flüssigen /fließfähigen Waschmittelformulierungen verleiht.

Typische erfindungsgemäße Oligoester haben z.B. folgende Struktur:

$$X-(OC_3CH_7)_m(OC_2H_4)_n -[OC(=O)-R^1-C(=O)O-R^2]_n-OC(=O)-R^1-C(=O)O-(C_2H_4O)_n (C_3CH_7O)_m-X$$

oder

$$X-(OC_2H_4)_n(OC_3CH_7)_m-[OC(=O)-R^1-C(=O)O-R^2]_n-OC(=O)-R^1-C(=O)O-(C_2H_4O)_n(C_3CH_7O)_m-X$$

oder insbesondere

$$X-(OC_2H_4)_n(OC_3CH_7)_m-[OC(=O)-R^1-C(=O)O-R^2]_u-OC(=O)-R^1-C(=O)O-(C_3CH_7O)_m(C_2H_4O)_n-X$$

mit

$R^1$ = im wesentlichen 1,4-Phenylen
$R^2$ = im wesentlichen 1,2-Propyliden
X = C1- bis C6- Alkyl, insbesondere Methyl oder Ethyl
n = von 10 bis 50 insbesondere 12 bis 20
m = von 2 bis 10, vorzugsweise 2 bis 4 und
u = von 3 bis 10.

Die Angabe "bei Raumtemperatur" steht jeweils für Temperaturen von 15 bis 25 °C, insbesondere 20°C. Verbindungen im Sinne des Hauptanspruchs der vorliegenden Erfindung sind organische Verbindungen die neben Kohlenstoff, Wasserstoff und Sauerstoff nach Umsetzung, d.h. Einbau in das Oligomer, keine weiteren Atome aufweisen. Dies bedeutet etwa, dass die Dicarbonsäure-Verbindungen nach Einbau in den Oligoester neben Carboxyl-Gruppen, auch Carbonyl- oder Hydroxy-Gruppen tragen können, aber z.B. keine Sulfonyl- oder Halogengruppen aufweisen.

Als Dicarbonsäure-Verbindung (A) können aliphatische und/oder aromatische Dicarbonsäuren bzw. deren Ester oder Anhydride eingesetzt werden. Die Dicarbonsäure-Verbindungen weisen - bezogen auf die Dicarbonsäure bzw. Dicarbonsäure-Gruppevorzugsweise 3 bis 40 Kohlenstoffatome auf.

Aromatische Dicarbonsäure-Verbindungen können erfindungsgemäß insbesondere Terephthalsäure, Isophthalsäure, Phthalsäure, deren Mono- und Dialkylester mit $C_1$ bis $C_5$-Alkoholen, wie z.B. Dimethylterephthalat sein, wobei auch Gemische dieser Verbindungen möglich sind.

Beispiele für aliphatische Dicarbonsäure-Verbindungen sind Malonsäure-, Bernsteinsäure-, Fumarsäure-, Maleinsäure-, Glutarsäure-, Adipinsäure-, Pimelinsäure-, Korksäure-, Azelainsäure- und Sebacinsäure-dialkylester. Besonders bevorzugt werden Terephthalsäure, Isophthalsäure und Phthalsäure sowie deren Dimethyl-, Diethyl-, Dipropyl- und Dibutylester eingesetzt.

Als Diol-Verbindung (B) können erfindungsgemäß beispielsweise Ethylenglykol, 1,2- bzw. 1,3-Propylenglykol, Neopentylglykol, 1,2-Butylenglykol, 3-Methoxy-1,2-propylenglykol sowie deren Dimere und Trimere eingesetzt werden. Die Diol-Verbindung (B) weist vorzugsweise 2 bis 20 Kohlenstoffatome auf. Grundsätzlich sind auch Mischungen verschiedener Diole möglich. Bevorzugt ist der ausschließliche Einsatz von Propylenglykol.

Beispiele für Alkylenoxid-Anlagerungsprodukte (C) sind Anlagerungsprodukte von Ethylenoxid / Propylenoxid oder Ethylenoxid / Butylenoxid an aliphatische $C_1$- bis $C_6$- Mono-Alkohole wie Methanol, Ethanol, Propanol oder Butanol. Das Anlagerungsprodukt zeichnet sich durch eine 2-Blockstruktur aus.

Die Synthese der Oligoester kann in Form einer direkten Umsetzung aller Monomerbausteine in einem Schritt erfolgen, so dass statistische Polymere erhalten werden. Eine andere Herstellungsweise ist eine Mehrschrittsynthese z.B. derart, dass eine Vorkondensation verschiedener Bausteine erfolgt.

Grundsätzlich werden bei der Synthese Temperaturen von ca. 80 bis 350 °C und Drücke von Normaldruck bis kleiner 1 mbar eingestellt. Vorzugsweise wird die Kondensation in dem Temperaturbereich von 150 bis 280 °C in Gegenwart der üblichen Polykondensations- und Umesterungskatalysatoren durchgeführt. Dabei können die gewichtsmittleren Molmassen der erzeugten Polymere (Oligoester) gezielt eingestellt werden. Diese liegen vorzugsweise zwischen 1000 und 6000 g/mol.

Als Katalysatoren eignen sich in der Literatur beschriebene Verbindungen. Werden als Dicarbonsäure-Verbindung (A) die freien Dicarbonsäuren oder die Anhydride verwendet, so sind p-Toluolsulfonsäure und Zinn-organische Verbindungen die bevorzugten Katalysatoren. Für Dicarbonsäuxedialkylester als Dicarbonsäure-Verbindung (A) werden die üblichen Umsterungskatalysatoren, wie beispielsweise Zinkacetat, Mischungen aus Calciumacetat und Antimonoxid, Stannane oder Tetraalkoxytitanate, wie Titamtetraisobutanolat oder Titantetraisopropanolat eingesetzt.

Die Kondensation kann in Gegenwart von Antioxidantien durchgeführt werden, z.B. von substituierten Phenolen, wie beispielsweise 2,5-Di.tertiärbutylphenol, 2-Methylcyclohexyl-4,6-dimethylphenul, phosphoriger Säure oder anderen üblicherweise hierfür verwendeten Antioxidantien. Diese Verbindungen verhindern oxidative Verfärbungen der Polyester während der Kondensation.

Soweit die Farbe der erfindungsgemäßen Oligoester weiterhin nicht zufriedenstellend ist, können diese einer Nachbehandlung unterzogen werden. Eine übliche Nachbehandlung ist beispielsweise eine Bleichung mit Wasserstoffperoxid, die zu einer deutlichen Farbaufhellung führt.

Die erfindungsgemäßen Oligoester können in unterschiedlicher Weise in Wasch- und Reinigungsmitteln eingesetzt werden. Sie sind bei Raumtemperatur fließfähig und können ohne weitere Bearbeitung als Additiv in die Wasch- und Reinigungsmittelprodukte eingebracht werden. So können zum Beispiel die Oligoester durch Einrühren bzw. Eindispergieren in flüssige oder gelförmige Wasch- und Reinigungsmittel eingearbeitet werden.

Grundsätzlich können diese Verbindungen auch in Form einer Matrix eingesetzt werden. Unter Matrix ist hierbei die Abmischung der Oligoester mit z.B.

- nichtionischen Tensiden, wie z.B. Alkoholethoxylaten, Alkoholpropoxylaten, gemischten Alkoholalkoxylaten, Alkylpolyglucosiden, Glukoseamiden,
- Polyethylenglykolen, Polypropylenglykolen, gemischten Polyalkylenglykolen,
- Lösungsmitteln wie Isopropanol, Propylenglykol, Glykolether, Wasser etc.

zu verstehen. Durch die Abmischung bzw. die Konfektionierung mit anderen Produkten können z.B. noch besser fließfähige Produkte niedriger Viskosität erhalten werden.

Die erfindungsgemäßen Oligoester können auch auf Trägermaterialien, wie z.B. Zeolithe, Phosphate, Citrate, Natriumsulfat, etc. z.B. durch Versprühen aufgebracht werden und dadurch z.B. in rieselfähige pulverformige Compounds überführt werden.

Derartige Compounds können vorteilhaft in pulverförmigen, granulatförmigen sowie extrudierten Wasch- und Reinigungsmitteln eingesetzt werden. Dabei erweist es sich als vorteilhaft, Produkte einzusetzen, die generell Formulierungsbestandteil der Wasch- und Reinigungsmittelformulierungen darstellen.

Die erfindungsgemäßen Oligoester können in einer Vielzahl von Wasch- und Reinigungsmitteln eingesetzt werden. Die Einsatzkonzentration an Oligoester in den Formulierungen beträgt vorzugsweise 0,01 bis 20 Gew.%, besonders bevorzugt 0,1 bis 5 Gew.%. Durch den Zusatz der erfindungsgemäßen Produkte wird die Wasch- und Reinigungsleistung gesteigert und die Neigung zur Wiederanschznutzung der gereinigten Oberfläche verringert.

Dabei zeichnen sich die erfindungsgemäßen Oligoester neben ihrer Soil-Release und Soil-Repellent Performance insbesondere durch ihre Unterstützung der Primärwaschleistung aus.

Die Oligoester können als Additive in textilen Reinigungsmitteln, wie Vollwaschbumitteln, Feinwaschmitteln, Gardinenwaschmitteln, Colorwaschmitteln, Teppichreinigungsmitteln, Imprägniermitteln, Vorbehandlungs- und Nachbehandlungsmittel etc. eingesetzt werden.

Darüber hinaus können die Oligoester auch in Wäscheweichspülmitteln eingesetzt werden. Der Zusatz der Produkte zu den Wäscheweichspülern, die üblicherweise hauptsächlich kationische Tenside enthalten, verbreitert das Wirkungsspektrum des Weichspülproduktes, da die kationischen Tenside bevorzugt auf natürlichen Textilfasern, wie z.B. Baumwollgewebe, wirken. Die erfindungsgemäßen Oligoester erweitern das Wirkungsspektrum auf synthetische Fasermaterialien, insbesondere polyesterhaltigen Gewebearten. Die nichtionischen Oligoester weisen eine gute Verträglichkeit mit kationischen Weichspülerrohstoffen auf und können daher gemeinsam in der Wäschenachbehandlung und Wäschepflege eingesetzt werden.

Neben der Reinigung von Fasern sind die erfindungsgemäßen Oligoester insbesondere zur Reinigung von harten Oberflächen in Haushalt und Gewerbe geeignet.

Bei den Reinigungsmitteln kann es sich um stark schäumende Einstellungen handeln, oder um schaumarme Reinigungsmittel, wie sie häufig in technischen Reinigerprodukten vorliegen. Die Formulierungen können zur manuellen und maschinellen Reinigung verwendet werden.

Neutrale Formulierungen werden z.B. als Allzweckreiniger im Haushalt oder zur Reinigung von Fahrzeugen verwendet. Saure Einstellungen, die u.a. kalklösende Wirksamkeit haben, werden z.B. im Sanitärbereich oder als Badreiniger eingesetzt, während alkalische Formulierungen beispielsweise als Universal-, Fußboden- und Glasreiniger Anwendung finden. Vorzugsweise beträgt der pH-Wert der Zusammensetzung bzw. Formulierung, in der die erfindungsgemäßen Oligoester eingesetzt werden, 3 bis 9.

Weitere Beispiele für den Einsatz der erfindungsgemäßen Oligoester in Reinigungsmitteln für harte Oberflächen sind z.B. Reiniger für Kunststoffoberflächen, Computergehäusereiniger, Auto- und Felgenreiniger, LKW-Planenreiniger, flüssige, gelförmige und blockförmige WC-Reiniger und Möbelpolituren.

**Beispiel**

In einem 2 1 Mehrhalskolben mit Glasrührer, Heizbad, Schutzgaseinleitung, Destillationsaufsatz, Füllkörperkolonne, Destillationsbrücke, Vakuumverteiler, Destillationskolben, Kühlfalle und Innenthermometer wurden insgesamt

- 827 g (ca. 0.94 mol) eines Block-Polyethylen-/p ropylenglykolmonomethylethers $(CH_3O(EO)_n(PO)_m-H)$ mit m = 4 und n = 14 mit einem gewichtsmittleren Molekulargewicht von ca. 880 g/mol,
- 194 g (1 mol) Dimethylterephthalat,
- 152 g (2 mol) 1,2-Pxopylenglykol,

1,0 g 2,6-Di-tert.-butyl-p-kresol (Ionol® von Shell) sowie 1 ml Tetraisopropylorthotitanat unter Schutzgas vorgelegt.

Die Reaktionsmischung wurde langsam bis auf Temperaturen von 150 bis 220 °C aufgeheizt und das gebildete Methanol aufgefangen. Nachdem der größte Teil der theoretisch zu erwartenden Methanolmenge aufgefangen war, wurde die Reaktionsmischung abgekühlt, die Kolonne ausgebaut, Vakuum angelegt und die Mischung wieder bis auf maximal 230°C aufgeheizt. Das bei der Reaktion nicht umgesetzte Diol-/Methanolgemisch wurde dabei als Destillat aufgefangen.

Nachdem der Oligoester eine Hydroxylzahl von ca. 10 mg KOH/g Substanz erreicht hatte, wurde die Reaktion abgebrochen. Das Produkt lag als gelbes, niedrigviskoses Öl vor.

**Vergleichsbeispiel**

In einem 2 1 Mehrhalskolben mit Glasrührer, Heizbad, Schutzgaseinleitung, Destillationsaufsatz, Füllkörperkolonne, Destillationsbrücke, Vakuumverteiler, Destillationskolben, Kühlfalle und Innenthermometer wurden insgesamt

- 883 g (2,0 mol) Polyethylenglykolmonomethylether mit einem gewichtsmittleren Molekulargewicht von ca. 440 g/mol (MARLIPAL® 1/12 der Sasol Germany GmbH), 534 g (2,75 mol) Dimethylterephthalat,
- 227,9 g (2,5 mol) Glycerin,
- 68,3 g (1,1 mol) Monoethylenglykol,

1,0 g 2,6-Di-tert.-butyl-p-kresol (Ionol® von Shell) sowie 1 ml Tetraisopropylorthotitanat unter Schutzgas vorgelegt.

Die Reaktionsmischung wurde langsam bis auf Temperaturen von 150 bis 220 °C aufgeheizt und das gebildete Methanol aufgefangen. Nachdem der größte Teil der theoretisch zu erwartenden Methanolmenge aufgefangen war, wurde die Reaktionsmischung abgekühlt, die Kolonne ausgebaut, Vakuum angelegt und die Mischung wieder bis auf maximal 230°C aufgeheizt. Das bei der Reaktion nicht umgesetzte Diol-/Polyolgemisch wurde dabei als Destillat aufgefangen.

Nachdem der Oligoester eine Hydroxylzahl von ca. 60 mg KOH/g Substanz erreicht hatte, wurde die Reaktion abgebrochen. Das Produkt lag als gelbes, niedrigviskoses Öl vor.

**Primärwaschleistung**

Zur Verifizierung der Primärwaschleistung der erfindungsgemäßen Oligoester wurden Waschversuche in einer haushaltüblichen Waschmaschine durchgeführt. Folgende Prüfgewebe der WFK wurden für die Waschteste eingesetzt:

| WEL Nummer | Gewebebezeichnung | Anschmutzung |
|---|---|---|
| 7962 | MG 20 D | Pigment / Hautfett |
| 7914 | MG 20 B | Pigment / Olivenöl |
| 7915 | MG 20 PPM | Pigment / Pflanzenfett / Milch |
| 7916 | MG 20 LI | Rotwein |
| 7917 | MG 20 LS | Lippenstift |
| 7918 | MG 20 MU | Make up |
| 7919 | MG 20 GM | Motoröl, gebraucht |
| 7270 | PE 30 D | Pigment / Hautfett |

Folgende Flüssigwaschnüttelfonnuherungen kamen zum Einsatz:

| Flüssigformulierung | A | B | C |
|---|---|---|---|
| Lineares Alkylbenzolsulfonat (50%ig) | 20 | 20 | 20 |
| C12-C15 Fettalkoholethoxylat (7 EO) | 10 | 10 | 10 |
| Kokosfettsäure | 12 | 12 | 12 |
| KOH (50%ig) | Ca. 6,05 | Ca. 6,05 | Ca. 6,05 |
| Natriumcitrat-Dihydrat | 3 | 3 | 3 |
| Phosphonat | 2 | 2 | 2 |
| 1,2-Pxopylenglykvl | 5 | 5 | 5 |
| Ethanol | 3 | 3 | 3 |
| Oligoester gemäß Beispiel | - | 0,7 | 1,0 |
| VE-Wasser zu 100% | 38,95 | 37,95 | 37,45 |
| Viskosität bei 25 °C | 134 mPas | 126 mPas | 132 mPas |
| pH-Wert | 8,5 | 8,5 | 8,5 |

Waschbedingungen:

| | |
|---|---|
| Waschmaschine: | Waschmaschinen Miele Novotronic W985 datacolor Spektraflash SF 450 |
| Waschtemperatur: | 40 °C |
| Präfkonzentration | 75 g pro Waschgang |
| Prüfbedingungen: | Wasserhärte 13°dH |
| Ballastgewebe / Programm | 3,5kg / Koch- u. Buzttwäsche |
| Waschflotte | ca. 12 L |
| Waschprogramm: | Hauptwaschgang ohne Vorwäsche |

Durchführung der Waschversuche:

Die Waschtrommel der jeweiligen Waschmaschine wurde mit je 3,5 kg Balastgewebe beschickt. Je Anschmutzung wurden je 2 Testläppchen der WFK verwendet. Diese wurden mit der testverschmutzten Seite nach oben auf ein weißes Baumwollhandtuch genäht. Dieses wurde in die Maschinen zu dem Balastgewebe gegeben.

Die Waschversuche wurden unter den oben genannten Bedingungen durchgeführt. Danach erfolgte die Messung des Waschwertes mit Hilfe eines Spektralphotometers (Datacolor Spektraflash SF 450). Der Waschwert gibt die Aufhellung eines gewaschenen Gewebes im Verhältnis zu einem testverschmutzten und einem ungewaschenen Gewebe in % an. Der Waschwert wird nach folgender Gleichung ermittelt:

$$\% \text{ Waschwert} = \frac{R_a - R_b}{R_c - R_b} \times 100$$

$R_a$ = Remission des gewaschenen Gewebes
$R_b$ = Remission des testverschmutzten Gewebes
$R_c$ = Remission des unverschmutzten Gewebes

Die Remission wurde mit dem Spektralphotometer bei einer Wellenlänge von 360 nm bis 750 nm bestimmt. Die Auswertung erfolgte mit dem Programm "Washtest". Das einzelne Läppchen wurde insgesamt 8x gemessen und der Mittelwert als Waschwert bestimmt.

Wie den Ergebnissen dieser Waschversuche zu entnehmen ist (siehe Tabelle) führt der Zusatz der erfindungsgemäßen Oligoester zu einer signifikanten Steigerung der Primärwaschleistung.

Untersuchung der chemischen Laizerstabilität der Oligoester

Die Oligoester gemäß o.g. Beispiel bzw. Vergleichsbeispiel wurden mit einer Konzentration von 1 % (Aktivsubstanz) in einer handelsüblichen Flüssigwaschmittelformulierung mit einem pH-Wert von 8.5 eingesetzt.

Die Testgewebe (Polyester bzw. Baumwoll/Polyestergewebe der Wäschereiforschungsanstalt Krefeld, WfK) wurden zuerst mit der handelsüblichen Formulierung gewaschen und nach dem Trocknen die Remissionswerte gemessen. Anschließend wurden sie mit jeweils 5 Tropfen gebrauchtem Motoröl angeschmutzt. Nach einer Einwirkzeit von ca. 18 h wurde die Remission der angeschmutzten Gewebestücke ermittelt. Nach einem erneuten Waschgang mit den entsprechenden Formulierungen wurden die Remissionswerte zum Schluß noch einmal bestimmt. Gewaschen wurde in Lini-Test-Labormaschinen.

Waschbedingungen:

| Waschtemperatur | 40°C |
| Waschdauer | 30 Minuten |
| Wasserhärte | 13° dH |
| Waschmittelkonzentration | 5 g t.q./l |
| Flottenverhältnis | ca. 1 : 83 |

Für die Berechnung der Aufhellung in % wurde die folgende Formel verwendet:

$$\text{Aufhellung [\%]} = \frac{(R_3 - R_2)}{(R_1 - R_2)} \times 100$$

$R_1$ = Remission des gewaschenen Gewebes
$R_2$ = Remission des ölverschmutzten Gewebes
$R_3$ = Remission des erneut gewaschenen ölverschmutzten Gewebes

Die Waschversuche wurden nach 4 Wochen mit den gleichen Formulierungen wiederholt. In dieser Zeit wurden die nicht-additivierten sowie mit Oligoester additivierten Formulierungen bei Raumtemperatur sowie bei 40 °C gelagert.

Folgende Oligoester wurden untersucht:

| Oligoester gemäß Beispiel: | Aktivgehalt 1 Gew.% |
| Oligoester gemäß Vergleichsbeispiel: | Aktivgehalt 1 Gew.% |

| Waschmittelformulierung | Ölablösung in [%] an Misch-gewebe | Ölablösung in [%] an Polyester |
| --- | --- | --- |
| Flüssigwaschmittelformulierung (Standard, ohne Oligoester) | 12 | 13 |
| + 1 Gew.% Oligoester gemäß Beispiel; sofort nach Dotierung | 25 | 31 |
| + 1 Gew.% Oligoester gemäß Beispiel; nach 4 Wochen Lagerung bei Raumtemperatur | 24 | 32 |
| + 1 Gew.% Oligoester gemäß Beispiel; nach 4 Wochen Lagerung bei 40 °C | 20 | 28 |

Tabelle fortgesetzt

| Waschmittelformulierung | Ölablösung in [%] an Misch-gewebe | Ölablösung in [%] an Polyester |
|---|---|---|
| + 1 Gew.% Oligoester gemäß Vergleichsbeispiel; sofort nach Dotierung | 22 | 28 |
| + 1 Gew.% Oligoester gemäß Vergleichsbeispiel; nach 4 Wochen Lagerung bei Raumtemperatur | 18 | 25 |
| + 1 Gew.% Oligoester gemäß Vexgleichsbeispiel; nach 4 Wochen Lagerung bei 40 °C | 12 | 15 |

In Analogie zu obigen Untersuchungen wurden die Oligoester gemäß Beispiel bzw. Vergleichsbeispiel in der selben handelsüblichen Flüssigwaschmittelformulierung eingesetzt, wobei der pH-Wert der Formulierung gezielt von 8.5 auf 9.0 durch Zugabe von verdünnter KOH erhöht wurde.

| Waschmittelformulierung | Ölablösung in [%] an Mischgewebe | Ölablösung in [%] an Polyester |
|---|---|---|
| Flüssigwaschmittelformulierung (ohne Oligoester) | 13 | 9 |
| + 1 Gew.% Oligoester gemäss Bsp.; sofort nach Dotierung | 27 | 39 |
| + 1 Gew.% Oligoester gemäss Bsp.; nach 2 Wochen Lagerung bei 40 °C | 26 | 29 |
| + 1 Gew.% Oligoester gemäss Bsp.; nach 4 Wochen Lagerung bei 40 °C | 20 | 20 |
| + 1 Gew.% Oligoester gemäss Vergleichsbeispiel; sofort nach Dotierung | 30 | 40 |
| + 1 Gew.% Oligoester gemäss Vergleichs beispiel; nach 2 Wochen Lagerung bei 40 °C | 17 | 18 |
| + 1 Gew.% Oligoester gemäss Vergleichsbeispiel; nach 4 Wochen Lagerung bei 40 °C | 14 | 11 |

Wie aus den Ergebnissen der Waschversuche hervorgeht, zeichnet sich der erfindungsgemäße Oligoester gemäß oben genanntem Beispiel durch eine deutlich bessere Hydrolysestabilität und somit verbesserte chemische Stabilität in der Flüssigformulierung aus.

## Einzelergebnisse des Waschvermögen bei 40°C

| Waschmittel je 75g | Maschine Miele | Anschmutzung: Mischgewebe / Polyester | | | | | | | |
|---|---|---|---|---|---|---|---|---|---|
| | | Pigment / Hautfett 20 D WEL 7962 | Pigment / Olivenöl 20 B WEL 7914 | Pigment / Pflanzenfett / Milch 20 PPM WEL 7915 | Rotwein 20 LI WEL 7916 | Lippenstift 20 LS WEL 7917 | Make up 20 MU WEL 7918 | Gebr. Motoröl 20 GM WEL 7919 | Pigment / Hautfett 30 D WEL 7270 |
| Ansatz A | W 985/1 | 74,1 ± 0,4 | 14,2 ± 0,1 | 67,2 ± 1,0 | 52,2 ± 0,1 | 86,3 ± 0,2 | 80,4 ± 0,1 | 14,6 ±0,1 | 60,5 ± 0,1 |
| | W 985/1 | 73,9 ± 0,1 | 14,7 ± 0,6 | 68,1 ± 0,8 | 52,6 ± 0,3 | 88,0 ± 0,1 | 79,9 ± 0,1 | 13,0 ± 0,1 | 59,2 ± 0,1 |
| | W 985/2 | 73,9 ± 0,1 | 14,2 ± 0,2 | 65,9 ± 0,6 | 51,0 ± 0,1 | 87,8 ± 0,1 | 79,5 ± 0,2 | 13,8 ± 0,3 | 58,0 ± 0,1 |
| | W 985/2 | 73,2 ± 0,1 | 14,9 ± 0,3 | 65,2 ± 0,9 | 51,7 ± 0,1 | 86,2 ± 0,1 | 77,5 ± 0,1 | 13,5 ± 0,2 | 57,7 ± 0,2 |
| | W 985/3 | 74,4 ± 0,1 | 15,0 ± 0,4 | 66,5 ± 1,0 | 48,0 ± 0,2 | 87,0 ± 0,1 | 78,9 ± 0,1 | 14,0 ± 0,1 | 58,8 ± 0,2 |
| | W 985/3 | 75,1 ± 0,1 | 15,7 ± 0,2 | 66,8 ± 0,7 | 49,2 ± 0,4 | 88,1 ± 0,2 | 79,1 ± 0,1 | 13,4 ± 0,1 | 57,9 ± 0,2 |
| Ansatz B | W 985/4 | 82,0 ± 0,1 | 19,0 ± 0,1 | 70,3 ± 0,8 | 50,5 ± 0,5 | 91,4 ± 0,1 | 81,0 ± 0,1 | 18,6 ± 0,2 | 70,8 ± 0,3 |
| | W 985/4 | 83,2 ± 0,2 | 21,3 ± 0,4 | 70,8 ± 0,2 | 51,2 ± 0,2 | 91,9 ± 0,1 | 81,1 ± 0,1 | 18,9 ± 0,1 | 71,6 ± 0,3 |
| | W 985/5 | 83,1 ± 0,1 | 19,6 ± 0,1 | 71,8 ± 0,2 | 50,0 ± 0,7 | 91,0 ± 0,3 | 80,7 ± 0,1 | 20,6 ± 0,2 | 71,6 ± 0,1 |
| | W 985/5 | 81,4 ± 0,1 | 20,0 ± 0,1 | 72,4 ± 0,6 | 51,2 ± 1,0 | 91,9 ± 0,1 | 80,0 ± 0,1 | 19,4 ± 0,3 | 70,0 ± 0,2 |
| | W 985/6 | 81,7 ± 0,1 | 19,3 ± 0,2 | 72,5 ± 0,7 | 48,0 ± 0,5 | 91,7 ± 0,1 | 81,1 ± 0,1 | 19,0 ± 0,3 | 69,1 ± 0,5 |
| | W 985/6 | 83,1 ± 0,1 | 19,1 ± 0,1 | 72,9 ± 0,2 | 48,8 ± 0,5 | 91,2 ± 0,1 | 80,1 ± 0,1 | 19,1 ± 0,2 | 70,1 ± 0,2 |
| Ansatz C | W 985/1 | 81,0 ± 0,3 | 21,8 ± 0,4 | 72,1 ± 0,3 | 45,6 ± 0,1 | 91,1 ± 0,1 | 80,3 ± 0,1 | 20,4 ± 0,1 | 72,5 ± 0,2 |
| | W 985/1 | 82,5 ± 0,2 | 22,6 ± 0,5 | 71,7 ± 0,1 | 46,1 ± 0,1 | 91,7 ± 0,2 | 81,1 ± 0,1 | 20,0 ± 0,3 | 72,1 ± 0,1 |
| | W 985/2 | 83,8 ± 0,1 | 21,0 ± 0,6 | 71,4 ± 0,1 | 45,1 ± 0,2 | 90,9 ± 0,1 | 81,7 ± 0,1 | 22,1 ± 0,1 | 73,1 ± 0,1 |
| | W 985/2 | 83,9 ± 0,4 | 21,4 ± 0,4 | 70,4 ± 0,9 | 45,9 ± 0,1 | 91,3 ± 0,1 | 81,0 ± 0,1 | 20,2 ± 0,2 | 71,1 ± 0,5 |
| | W 985/3 | 82,1 ± 0,1 | 21,7 ± 0,2 | 69,4 ± 0,2 | 49,1 ± 0,2 | 91,1 ± 0,1 | 80,2 ± 0,1 | 21,0 ± 0,1 | 73,6 ± 0,3 |
| | W 985/3 | 81,9 ± 0,1 | 21,9 ± 0,6 | 69,5 ± 0,3 | 48,2 ± 0,8 | 90,0 ± 0,1 | 80,9 ± 0,1 | 23,0 ± 0,4 | 72,2 ± 0,4 |

EP 1 661 933 A1

**Patentansprüche**

1. Bei Raumtemperatur fließfähige, amphiphile und nichtionische Oligoester, **dadurch gekennzeichnet**, d a s s die Oligoester durch Umsetzung von

   (A) 15 bis 50 mol% einer oder mehrerer Dicarbonsäure-Verbindungen,
   (B) 4 bis 70 mol% einer oder mehrerer Diolverbindungen und
   (C) 5 bis 60 mol% eines oder mehrerer wasserlöslicher Alkylenoxid-Anlagerungsprodukte mindestens zweier unterschiedlicher Alkylenoxid-Einheiten an einen C1- bis C6-Alkohol im Molverhältnis 4 bis 100 Mol Alkylenoxid zu 1 Mol Alkohol herstellbar sind und das Alkylenoxid-Anlagerungsprodukt eine 2-Blockstruktur aufweist aus einem C2- Alkylenoxid-Block und entweder einem C3- Alkylenoxid-Block oder einem C4-Alkylenoxid-Block.

2. Oligoester gemäß Anspruch 1 herstellbar unter Verwendung von 25 bis 40 mol% der Dicarbonsäure-Verbindung,

3. Oligoester gemäß einem oder mehreren der vorhergehenden Ansprüche herstellbar unter Verwendung von 15 bis 40 mol% des Alkylenoxid - Anlagerungsproduktes.

4. Oligoester gemäß einem oder mehreren der vorhergehenden Ansprüche herstellbar unter Verwendung des Alkylenoxid-Anlagerungsproduktes aufweisend ein Molverhältnis von 10 bis 50 Mol Alkylenoxid zu 1 Mol Alkohol.

5. Oligoester gemäß einem oder mehreren der vorhergehenden Ansprüche, d a - d u r c h **gekennzeichnet** , das s die Dicarbonsäure-Verbindung zu zumindest 50 mol%, vorzugsweise zu zumindest 90 mol% und insbesondere ausschließlich eine Phthalsäure-Verbindung ist und die Phthalsäure-Verbindung ausgewählt ist aus der Gruppe Pthalsäure, Terephthalsäure und/oder Isoterephthalsäure, insbesondere Terephthalsäure.

6. Oligoester gemäß einem oder mehreren der vorhergehenden Ansprüche, d a - durch **gekennzeichnet**, d a s s die Diolverbindungen zu zumindest 50 mol%, vorzugsweise zu zumindest 90 mol% und insbesondere ausschließlich 1,2-Propylenglykol ist.

7. Oligoester gemäß einem oder mehreren der vorhergehenden Ansprüche, d a - durch **gekennzeichnet**, d a s s das Alkylenoxid-Anlagerungsprodukt durch Anlagerung

   • von Propylenoxid und nachfolgend Ethylenoxid oder insbesondere
   • von Ethylenoxid und nachfolgend Propylenoxid oder
   • von Butylenoxid und nachfolgend fithylenoxid oder insbesondere
   • von Ethylenoxid und nachfolgend Butylenoxid

   an einen C1- bis C6- Mono-Alkohol, vorzugsweise Methanol, dargestellt ist.

8. Oligoester gemäß einem oder mehreren der vorhergehenden Ansprüche, d a - d u r c h **gekennzeichnet**, d a s s das Alkylenoxid-Anlagerungsprodukt ein Anlagerungsprodukt von 2 bis 4 mol Propylenoxid und 10 bis 20 mol Ethylenoxid an den C1- bis C6- Mono-Alkohol, vorzugsweise Methanol, ist.

9. Oligoester gemäß einem oder mehreren der vorhergehenden Ansprüche, d a - durch **gekennzeichnet**, d a s s die Dicarbonsäure-Verbindung bzw. das entsprechend endständig substituierte Teilpolymer durch Veresterung / Umesterung mit dem Alkylenoxid-Anlagerungsprodukt verknüpft ist, wobei die Verknüpfung mit einer Propylenoxid-Einheit oder einer Butylenoxid-Einheit, insbesondere einer Propylenoxid-Einheit vorgenommen ist.

10. Oligoester gemäß einem oder mehreren der vorhergehenden Ansprüche, d a - d u r c h **gekennzeichnet** , d a s s das Alkylenoxid-Anlagerungsprodukt ein gewichtsmittleres Molekulargewicht von kleiner 1000 g/mol aufweist.

11. Oligoester gemäß einem oder mehreren der vorhergehenden Ansprüche, d a - d u r c h **gekennzeichnet**, dass das Molekulargewicht des Oligoesters zwischen 1000 und 6000 g/mol beträgt, vorzugsweise zwischen 1500 und 4000 g/mol.

12. Bei Raumtemperatur flüssige oder fließfähige Wasch- bzw. Reinigungsmittelformulierungen enthaltend 0,1 bis 5 Gew.% der erfindungsgemäßen Oligoester gemäß einem der Ansprüche 1 bis 11.

13. Bei Raumtemperatur flüssiges oder fließfähiges Konzentrat enthaltend 50 bis 95 Gew% Oligoester gemäß einem der Ansprüche 1 bis 11 und weiterhin eine oder mehrere der nachfolgend genannten Komponenten: nichtionische Tenside, Polyalkylenglykole, Alkylenglykole und Wasser, wobei das Konzentrat vorzugsweise einen pH-Werte von größer 8 oder sogar größer 8,5 aufweist.

14. Verwendung der Oligoester gemäß einem oder mehreren der Ansprüche 1 bis 11 in Vollwaschmitteln, Feinwaschmitteln, Colorwaschmitteln, Gardinenwaschmitteln, Wäscheweichspülern sowie Teppichreinigungs- und Imprägniermitteln, Vorbehandlungs- und Nachbehandlungsmittel, insbesondere solchen die pH-Werte von Grö-βer 8 oder sogar größer 8,5 aufweisen.

15. Verwendung der Oligoester gemäß einem oder mehreren der Ansprüche 1 bis 11 in pulver- bzw. granulatförmigen Waschmittelformulierungen, durch Versprühen des Oligoesters auf einen Träger.

16. Verwendung der Oligoester gemäß einem oder mehreren der Ansprüche 1 bis 11 als Additive in bei Raumtemperatur flüssigen oder fließfähigen Reinigerformulierungen, insbesondere solchen zur Reinigung harter Oberflächen

17. Verwendung der Oligoester gemäß einem oder mehreren der Ansprüche 1 bis 11 in Haarbehandlungsmitteln.

**Europäisches Patentamt**

## EUROPÄISCHER RECHERCHENBERICHT

**Nummer der Anmeldung**

EP 05 02 5645

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 0 185 427 A (THE PROCTER & GAMBLE COMPANY) 25. Juni 1986 (1986-06-25) <br> * Seite 5, Zeile 1 - Zeile 34 * <br> * Seite 6, Zeile 25 - Seite 7, Zeile 36 * <br> * Seite 12, Zeile 1 - Zeile 24; Beispiele * <br><br> ----- | 1-17 | C08G63/672 |
| A | WO 02/18474 A (SASOL GERMANY GMBH; KOCH, HERBERT; SCHOENKAES, UDO) 7. März 2002 (2002-03-07) <br> * Seite 3, Zeile 27 - Seite 4, Zeile 6 * <br> * Seite 6, Zeile 7 - Seite 7, Zeile 2; Beispiele * <br><br> ----- | 1-17 | |
| A | DE 44 17 686 A1 (HENKEL KGAA, 40589 DUESSELDORF, DE) 23. November 1995 (1995-11-23) <br> * Seite 2, Zeile 51 - Seite 3, Zeile 38 * <br> ----- | 1-17 | |
| A | DE 198 26 356 A1 (CLARIANT GMBH) 16. Dezember 1999 (1999-12-16) <br> * Seite 3, Zeile 7 - Zeile 48 * <br> ----- | 1-17 | RECHERCHIERTE SACHGEBIETE (IPC) <br><br> C08G <br> C11D |

Der vorliegende Recherchenbericht wurde für alle Patentansprüche erstellt

| Recherchenort | Abschlußdatum der Recherche | Prüfer |
|---|---|---|
| München | 2. März 2006 | Komenda, C |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**          EP 05 02 5645

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-03-2006

| Im Recherchenbericht angeführtes Patentdokument | | Datum der Veröffentlichung | Mitglied(er) der Patentfamilie | | Datum der Veröffentlichung |
|---|---|---|---|---|---|
| EP 0185427 | A | 25-06-1986 | AU | 580122 B2 | 05-01-1989 |
| | | | AU | 5153685 A | 26-06-1986 |
| | | | CA | 1315286 C | 30-03-1993 |
| | | | DE | 3585505 D1 | 09-04-1992 |
| | | | DK | 599785 A | 22-06-1986 |
| | | | FI | 855117 A | 22-06-1986 |
| | | | GB | 2168989 A | 02-07-1986 |
| | | | GR | 853077 A1 | 17-04-1986 |
| | | | HK | 50394 A | 27-05-1994 |
| | | | IE | 58784 B1 | 17-11-1993 |
| | | | JP | 2588507 B2 | 05-03-1997 |
| | | | JP | 61209299 A | 17-09-1986 |
| | | | JP | 2596409 B2 | 02-04-1997 |
| | | | JP | 7166192 A | 27-06-1995 |
| | | | KR | 9308481 B1 | 07-09-1993 |
| WO 0218474 | A | 07-03-2002 | AT | 266691 T | 15-05-2004 |
| | | | AU | 9162101 A | 13-03-2002 |
| | | | CN | 1462288 A | 17-12-2003 |
| | | | DE | 10043604 A1 | 28-03-2002 |
| | | | DE | 10193622 D2 | 02-10-2003 |
| | | | DK | 1313787 T3 | 16-08-2004 |
| | | | EP | 1313787 A1 | 28-05-2003 |
| | | | ES | 2218453 T3 | 16-11-2004 |
| | | | HU | 0301702 A2 | 28-08-2003 |
| | | | JP | 2004507597 T | 11-03-2004 |
| | | | US | 2005101667 A1 | 12-05-2005 |
| DE 4417686 | A1 | 23-11-1995 | KEINE | | |
| DE 19826356 | A1 | 16-12-1999 | CN | 1239107 A | 22-12-1999 |
| | | | EP | 0964015 A1 | 15-12-1999 |
| | | | ES | 2209280 T3 | 16-06-2004 |
| | | | JP | 2000026492 A | 25-01-2000 |
| | | | US | 6153723 A | 28-11-2000 |

EPO FORM P0461

Für nähere Einzelheiten zu diesem Anhang : siehe Amtsblatt des Europäischen Patentamts, Nr.12/82